(19) Europäisches Patentamt
European Patent Office
Office européen des brevets

(11) Veröffentlichungsnummer: **0 419 408 A1**

(12) # EUROPÄISCHE PATENTANMELDUNG

(21) Anmeldenummer: **90810683.4**

(51) Int. Cl.[5]: **C07D 235/30**

(22) Anmeldetag: **11.09.90**

(30) Priorität: **18.09.89 CH 3388/89**

(43) Veröffentlichungstag der Anmeldung:
**27.03.91 Patentblatt 91/13**

(84) Benannte Vertragsstaaten:
**CH DE FR GB LI**

(71) Anmelder: **CIBA-GEIGY AG**
**Klybeckstrasse 141**
**CH-4002 Basel(CH)**

(72) Erfinder: **Ruf, Klaus, Dr.**
**Eckstrasse 24**
**W-7801 Bollschweil(DE)**

(54) **Herstellung von 2-Aminobenzimidazol.**

(57) Verfahren zur Herstellung von 2-Aminobenzimidazol durch Hydrolyse von Methyl-2-benzimidazolcarbamat mit 5 bis 20 Gew.%igem wässrigem Alkalihydroxid bei Temperaturen zwischen 80 und 100 °C und anschliessend Isolierung des erhaltenen Produkts nach an sich bekannten Methoden.

Dadurch ist es möglich 2-Aminobenzimidazol aus einem grosstechnischen Produkt mit einfachen, unbedenklichen Mitteln und mit einem sehr hohen Reinheitsgrad zu erhalten.

EP 0 419 408 A1

## HERSTELLUNG VON 2-AMINOBENZIMIDAZOL

Die vorliegende Anmeldung betrifft die Herstellung von 2-Aminobenzimidazol durch Hydrolyse von Methyl-2-benzimidazolcarbamat in verdünnter Alkalilauge.

Methyl-2-benzimidazolcarbamat, auch Carbendazim genannt, ist ein grosstechnisches Produkt, das als Fungizid Verwendung findet. Im Journal of Food Science and Technology 24 , (1987), 309-311, ist die alkalische Hydrolyse von Carbendazim mit konzentrierter (6,5 N) Natronlauge zu 2-Aminobenzimidazol für den analytischen Nachweis von geringen Mengen (2-10 mg) Carbendazim beschrieben. Dabei wird ausgeführt, dass diese Hydrolysemethode auf dem siedenden Wasserbad nicht quantitativ durchgeführt werden kann, sondern grössere Wärmezufuhr, wie z.B. Erwärmen auf einer Heizplatte oder auf offener Flamme, erfordert. Im Labormassstab führt die alkalische Hydrolyse mit 6,5 N Natronlauge bei Siedetemperatur in der Tat zu unvollständigen Umsetzungen.

Es ist nun gefunden worden, dass bei Durchführung der Hydrolyse von Carbendazim mit verdünntem wässrigem Alkalihydroxid bei Temperaturen zwischen 80 und 100° C 2-Aminobenzimidazol mit überraschend guten Ausbeuten erhalten werden kann.

Dadurch ist es möglich, 2-Aminobenzimidazol, das bekanntlich als Zwischenprodukt zur Herstellung von Pharmazeutika, Pflanzenschutzmitteln, Farbstoffen und Pigmenten Verwendung findet, aus einem grosstechnischen Produkt mit einfachen, unbedenklichen Mitteln und mit einem sehr hohen Reinheitsgrad vorteilhaft zu gewinnen.

Die vorliegende Erfindung betrifft demnach ein Verfahren zur Herstellung von 2-Aminobenzimidazol durch Hydrolyse von Methyl-2-benzimidazolcarbamat mit 5 bis 20 Gew.%igem wässrigem Alkalihydroxid bei Temperaturen zwischen 80 und 100° C und anschliessende Isolierung des erhaltenen Produkts nach an sich bekannten Methoden.

Als Alkalihydroxide kommen z.B. Lithiumhydroxid, bevorzugt Kaliumhydroxid und insbesondere Natriumhydroxid in Frage.

Im allgemeinen verwendet man 10 bis 19 %iges wässriges Alkalihydroxid, insbesondere aber 12 bis 17 und bevorzugt 14-15 %iges Natriumhydroxid.

Die Hydrolyse wird vorzugsweise bei Temperaturen zwischen 90 und 98° C, insbesondere zwischen 93 und 95° C bei einer bevorzugten Reaktionsdauer von 10 bis 20 Stunden durchgeführt. Die Hydrolysenzeiten variieren jedoch in Abhängigkeit von den Ansatzkonzentrationen.

Bei der Durchführung des erfindungsgemässen Verfahrens setzt man zweckmässig 2,05 bis 2,5, bevorzugt 2,1 bis 2,3, Mol Alkalihydroxid pro Mol Carbendazim ein.

Der Reaktionsablauf kann für das erfindungsgemässe Verfahren durch das folgende Formelschema wiedergegeben werden:

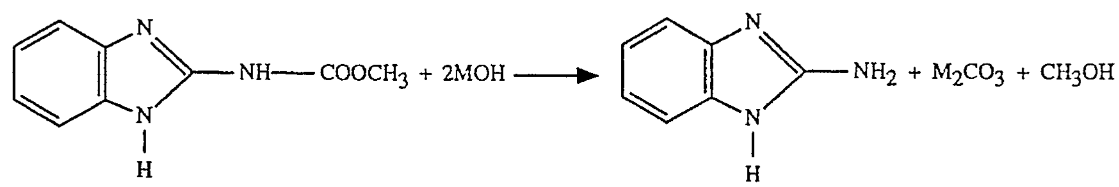

M = Li, K, Na

Die Hydrolyse lässt man im allgemeinen bei Normaldruck ablaufen.

Nach Beendigung der Reaktion kann das entstandene Methanol abdestilliert werden. Das nach dem Abkühlen auskristallisierte 2-Aminobenzimidazol kann dann abfiltriert, mit wenig Wasser gewaschen und getrocknet werden.

Durch die Bildung von $CO_2$ können bei der Reaktionstemperatur des erfindungsgemässen Verfahrens Schaumprobleme entstehen. Es empfiehlt sich deshalb ein herkömmliches Antischaummittel einzusetzen. Dazu eignen sich z.B. 2-Ethylhexanol, Cetylalkohol, hochmolekulare Glykole, gegebenenfalls im Gemisch mit Alkoholen oder anderen Glykolen, ferner Fettsäurepolyglykolester, Trialkylmelamine oder Silicone.

Um eine gute Rührbarkeit zu gewährleisten, ist es zweckmässig, das Carbendazim portionenweise, bevorzugt in zwei Portionen, z.B. die Hälfte vor und die andere Hälfte nach dem Erhitzen auf die gewünschte Hydrolysentemperatur, in die vorgelegte, das Antischaummittel enthaltende Alkalihydroxidlösung einzutragen.

Das nach dem erfindungsgemässen Verfahren erhaltene 2-Aminobenzimidazol zeichnet sich durch eine

sehr hohe Reinheit aus.

Die nachfolgenden Beispiele erläutern die Erfindung.

Beispiel 1:

In einem 5l-Sulfierkolben wird eine Lösung von 193,5 g NaOH in 1150 g Wasser (4,84 Mol: ca. 15 %ig) zusammen mit 4,15 g eines Antischaummittels auf Glykolbasis (®Surfynol der Air Products and Chem. Inc., Allentown P.A., USA) vorgelegt und unter Rühren auf 60°C erhitzt. Nach Zugabe von 210 g Carbendazim wird die Temperatur auf 93-95°C erhöht, dann werden weitere 210 g (insgesamt 2,14 Mol) Carbendazim eingetragen. Dies entspricht einem Molverhältnis NaOH:Carbendazim von 2,26:1. Man rührt 12 Stunden bei dieser Temperatur am Rückfluss (etwa bei 50 % Umsatz lässt die Schaumbildung nach. Danach kann die Temperatur auch auf 97-98°C erhöht werden). Sodann destilliert man das entstandene Methanol ab und lässt den Rückstand auf Raumtemperatur abkühlen. Das kristallisierte 2-Aminobenzimidazol wird abfiltriert und mit 650 g Wasser nachgewaschen. Man erhält 261,5 g (91,4 % d. Th.) 2-Aminobenzimidazol mit einem Schmelzpunkt von 225°C und einem Reinheitsgrad von 99,5 %. Durch Aufarbeitung der Mutterlauge kann die Ausbeute noch verbessert werden.

Beispiel 2:

Verfährt man wie in Beispiel 1 beschrieben, mit der einzigen Ausnahme, dass man anstelle der Natronlauge eine äquivalente Menge Kalilauge als 19 %ige wässrige Lösung (271 g KOH in 1150 g Wasser) verwendet, so erhält man 249,6 g (87,2 % d.Th.) 2-Aminobenzimidazol mit einem Reinheitsgrad von 97 %. Durch Aufarbeitung der Mutterlauge kann die Ausbeute noch verbessert werden.

Beispiel 3:

Verfährt man wie in Beispiel 1 beschrieben, setzt aber 12,4 %ige wässrige Natronlauge in einem Molverhältnis NaOH:Carbendazim von 2,05:1 ein, so erhält man 2-Aminobenzimidazol mit einer Ausbeute von 93,0% und einem Reinheitsgrad von 95,3 %.

Beispiel 4:

Verfährt man wie in Beispiel 1 beschrieben, setzt aber 16,5 %ige wässrige Natronlauge in einem Molverhältnis NaOH:Carbendazim von 2,5:1 ein, so erhält man 2-Aminobenzimidazol mit einer Ausbeute von 76,7 % und einem Reinheitsgrad von 94,1 %.

Beispiel 5:

Verfährt man wie in Beispiel 1 beschrieben, setzt aber 10 %ige wässrige Natronlauge in einem Molverhältnis NaOH:Carbendazim von 2,25:1 ein, so erhält man 2-Aminobenzimidazol mit einer Ausbeute von 91,0 % und einem Reinheitsgrad von 98,0 %.

Beispiel 6:

Verfährt man wie in Beispiel 1 beschrieben, aber bei 90°C statt bei 93-95°C und setzt 12,4 %ige Natronlauge im Molverhältnis NaOH:Carbendazim von 2,25:1 ein, so erhält man 2-Aminobenzimidazol mit einer Ausbeute von 92,0 % und einem Reinheitsgrad von 97,8 %.

## Ansprüche

1. Verfahren zur Herstellung von 2-Aminobenzimidazol durch Hydrolyse von Methyl-2-benzimidazolcarbamat mit 5 bis 20 Gew.%igem wässrigem Alkalihydroxid bei Temperaturen zwischen 80 und 100°C und anschliessende Isolierung des erhaltenen Produkts nach an sich bekannten Methoden.

2. Verfahren gemäss Anspruch 1, dadurch gekennzeichnet, dass ein 10 bis 19 %iges wässriges Alkalihydroxid verwendet wird.

3. Verfahren gemäss Anspruch 1, dadurch gekennzeichnet, dass ein 12 bis 17 %iges wässriges Natriumhydroxid verwendet wird.

4. Verfahren gemäss Anspruch 1, dadurch gekennzeichnet, dass die Hydrolyse bei Temperaturen zwischen 90 und 98°C erfolgt.

5. Verfahren gemäss Anspruch 1, dadurch gekennzeichnet, dass 2,05 bis 2,5 Mol Alkalihydroxid pro Mol Methyl-2-benzimidazolcarbamat verwendet werden.

6. Verfahren gemäss Anspruch 1, dadurch gekennzeichnet, dass die Hydrolyse in Gegenwart eines Antischaummittels durchgeführt wird.

7. Verfahren gemäss Anspruch 1, dadurch gekennzeichnet, dass das Methyl-2-benzimidazolcarbamat portionenweise in die vorgelegte, das Antischaummittel enthaltende Alkalihydroxidlösung eingetragen wird.

8. Verfahren gemäss Anspruch 1, dadurch gekennzeichnet, dass die Reaktionsdauer 10 bis 20 Stunden beträgt.

9. Verfahren gemäss Anspruch 1, dadurch gekennzeichnet, dass die Hydrolyse bei Normaldruck durchgeführt wird.

Europäisches Patentamt

# EUROPÄISCHER RECHERCHENBERICHT

Nummer der Anmeldung

**EP 90 81 0683**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | CHEMICAL ABSTRACTS, Band 108, Nr. 13, 28. März 1988, Seite 233, Spalte 2, Zusammenfassung Nr. 108003r, Columbus, Ohio, US; J.R. RANGASWAMY et al.: "Colorimetric method for the determination of carbendazim (MBC), benomyl and their degradative product 2-aminobenzimidazole", & J. FOOD. SCI. TECHNOL. 1987, 24(6), 309-11 * Zusammenfassung * ----- | | C 07 D 235/30 |
| | | | **RECHERCHIERTE SACHGEBIETE (Int. Cl.5)** |
| | | | C 07 D 235/00 |

Der vorliegende Recherchenbericht wurde für alle Patentansprüche erstellt

| Recherchenort | Abschlußdatum der Recherche | Prüfer |
|---|---|---|
| Den Haag | 29 November 90 | DE BUYSER I.A.F. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument